# EUROPÄISCHE PATENTSCHRIFT

(11) **EP 1 204 401 B1**
(45) Veröffentlichungstag und Bekanntmachung des Hinweises auf die Patenterteilung: **11.10.2006**
(21) Anmeldenummer: 00960403.4
(22) Anmeldetag: 08.08.2000
(51) Int. Cl.: A61Q 5/10, A61K 8/19

(54) **VERFAHREN ZUR OXIDATIVEN FÄRBUNG VON KERATINFASERN**
METHOD FOR OXIDATION DYEING OF KERATIN FIBRES
PROCEDE DE COLORATION OXYDATIVE DE FIBRES KERATINIQUES

(30) Priorität: 17.08.1999 DE 19938994
(43) Veröffentlichungstag der Anmeldung: 15.05.2002
(73) Patentinhaber: Henkel Kommanditgesellschaft auf Aktien, 40589 Düsseldorf (DE)
(72) Erfinder: STEPHAN, Hans-Oscar, 47269 Duisburg (DE); KLEEN, Astrid, 40699 Erkrath (DE); HELLER, Melita, 40591 Düsseldorf (DE); HÖFFKES, Horst, 40595 Düsseldorf (DE)
(86) Internationale Anmeldenummer: PCT/EP2000/007656
(87) Internationale Veröffentlichungsnummer: WO 2001/012143

(56) Entgegenhaltungen:
- EP-A- 0 148 681
- WO-A-87/01033
- DE-U- 29 805 893

## Beschreibung

Die Erfindung betrifft ein Verfahren zur oxidativen Färbung von Keratinfasern, bei dem eine wäßrige Zubereitung von Oxidationsfarbstoffvorprodukten zum Einsatz kommt, die vor der Einwirkung nicht mit einem Oxidationsmittel vermischt wird und unmittelbar vor oder während der Einwirkung auf die Keratinfaser mit einer feinteiligen, wasserunlöslichen Übergangsmetall-Verbindung in Kontakt gebracht wird, wodurch die Oxidation zum Farbstoff katalysiert und eine Färbung unter schonenden Bedingungen möglich gemacht wird.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, die unter dem Einfluß des atmosphärischen Sauerstoffs oder nach Zusatz eines Oxidationsmittels oxidiert und zum Farbstoff polymerisiert werden. Als Oxidationsfarbstoffvorprodukte eignen sich z.B. Phenole und aromatische Aminoverbindungen, die in Ortho- oder Paraposition eine weitere Amino- oder Hydroxylfunktion aufweisen. Weitere Oxidationsfarbstoffvorprodukte sind Heterocyclen wie z.B. Pyridine, Pyrazole, Chinoline, Indazole, Pyrimidine und Indole mit zwei oder mehr Amino und/oder Hydroxylgruppen, die miteinander in Konjugation treten und durch Oxidation Chinoide oder andere zu Farbstoffen polymerisierbare Zwischenstufen ausbilden können. Diese primären Oxidationsfarbstoff-Vorläufer werden auch Entwickler genannt. Die damit erzielbaren Färbungen lassen sich durch Zusatz von sogenannten sekundären Oxidationsfarbstoff-Vorläufern (Kupplern, color-modifier) verändern und in ihrer Vielfalt und Intensität erheblich steigern. Als Kuppler eignen sich z.B. Phenole und aromatische Amine mit einer weiteren Hydroxy-oder Aminogruppe in Meta-Position.

Die Oxidation der primären Oxidationsfarbstoff-Vorprodukte verläuft im alkalischen Medium rascher und vollständiger, daher werden handelsübliche Oxidationsfärbemittel so aufgebaut, daß der Färbeansatz einen pH-Wert von 8-10 aufweist. Auch die Gegenwart eines Oxidationsmittels, z.B. von Persulfaten. Chloriten oder bevorzugt von Wasserstoffperoxid führt zu einer rascheren und vollständigeren Oxidation als der bloße Zutritt von Luftsauerstoff. Daher kann man bei vielen Oxidationsfarbstoffen auf die Anwendung von Wasserstoffperoxid nicht verzichten - obwohl dies aus dermatologischen Gründen und zur Schonung der Keratinstruktur durchaus erwünscht wäre. Es ist zwar bekannt, daß die Oxidation der Oxidationsfarbstoffvorprodukte durch den Einsatz bestimmter Katalysatoren, z.B. durch Enzyme, Redox-Systeme, z.B. Jodide oder Chinone oder durch Metallkatalysatoren beschleunigt werden kann, die genannten Katalysatoren weisen aber bisher noch erhebliche Nachteile auf, so daß sich ihr Einsatz noch nicht durchgesetzt hat. So haben z.B. Oxidaseenzyme und Redoxsysteme eine verhältnismäßig schwache Wirkung gezeigt, so daß die im dermatologisch interessanten, neutralen pH-Bereich erzielten Färbeintensitäten bei vielen Farbstoffen unbefriedigend sind. Übergangsmetallkatalysatoren in gelöster oder komplexgebundener Form können in das Haar eindringen, so daß sie nach dem Färbevorgang nicht restlos ausgespült werden und im laufe der Zeit Haarschädigungen verursachen.

Es bestand daher die Aufgabe, ein Verfahren zur oxidativen Färbung von Keratinfasern zu entwickeln, bei dem die Oxidation der Oxidationsfarbstoffvorprodukte, d.h. die Entwicklung der Färbung im neutralen oder schwachsauren, für Haut und Haar schonenden pH-Bereich stattfindet oder bei dem der Einsatz von Oxidationsmitteln ganz unterbleiben kann, so daß keine Schädigung des Haars oder der Kopfhaut erfolgt.

Aus DE 298 05 893 U1 ist ein Mittel für die oxidative Färbung von Haaren bekannt, das als Katalysator eine geringe Menge eines feinverteilten Edelmetalls, z.B. Silber, enthält. Aus EP 235 217 B1 ist ein Verfahren zur oxidativen Färbung von Haaren bekannt, bei dem Mangandioxid in Pulverform als Oxidationskatalysator zum Einsatz kommt.

Eine Teilchengröße der eingesetzten Produkte ist nicht angegeben, handelsübliches Braunsteinpulver weist aber eine Teilchengröße von mehr als 1 Mikrometer (µm) auf.

Es wurde nun überraschend festgestellt, daß wasserunlösliche, nanoskalige Verbindungen der Übergangsmetalle, vorzugsweise die Oxide, die Entwicklung von Oxidationsfärbemitteln schon in Abwesenheit eines Oxidationsmittels bzw. in Gegenwart von Luftsauerstoff und bei niedrigen pH-Werten im neutralen oder schwach sauren Bereich ausreichend katalysieren.

Gegenstand der Erfindung ist daher die Verwendung wasserunlöslicher. feinteiliger Verbindungen der Metalle der 03. bis 12. Gruppe des periodischen Systems einschließlich der Lanthanide, vorzugsweise der Oxide mit einer Teilchengröße von weniger als 1000 nm, als Katalysatoren zur Farbentwicklung in Zubereitungen zur oxidativen Färbung der Haare ohne Züsatz von Oxidationsmitteln.

Diese Verwendung kann z.B. in der Weise erfolgen, daß man die katalytisch wirksamen Metallverbindungen in die Zubereitung der Oxidationsfarbstoffe einarbeitet und diese dann bis zur Anwendung auf den Fasern unter Luftabschluß aufbewahrt.

Bevorzugt wird die erfindungsgemäße Verwendung aber in der Weise erfolgen, daß man die Oxidationsfarbstoffvorprodukte und den Katalysator bis zur Anwendung getrennt hält und erst unmittelbar vor oder gleichzeitig mit der Einwirkung auf die Keratinfaser die oxidative Entwicklung einleitet.

Ein weiterer Gegenstand der vorliegenden Erfindung ist daher ein Verfahren zur oxidativen Färbung von Keratinfasern unter Verwendung einer wäßrigen Zubereitung von Oxidationsfarbstoff-Vorprodukten, die vor der Einwirkung auf die Fasern nicht mit einem Oxidationsmittel vermischt, dann mit dem Haar in Kontakt gebracht und nach einer Einwirkungszeit wieder ausgewaschen wird, wobei die Zubereitung unmittelbar vor oder gleichzeitig mit der Einwirkung auf die Keratinfasern mit einer wasserunlöslichen, feinteiligen Verbindung eines Metalls der 3. bis 12. Gruppe des periodischen Systems einschließlich der Lanthanide, vorzugsweise einem Oxid, mit einer Teilchengröße von weniger als 1000 nm in einer Menge von wenigstens 10 ppm, berechnet als Metall, bezogen auf den gebrauchsfertigen Färbeansatz, in Kontakt gebracht wird.

Das "in Kontakt bringen" kann dabei so erfolgen, daß die feinteiligen Metallverbindungen als Pulver oder als Dispersion in einem wäßrigen oder wassermischbaren Trägermedium der Zubereitung der Oxidationsfarbstoffvorprodukte zugemischt wird. Als wassermischbare Trägermedien eignen sich z.B. Glycerin, Propylenglycole, Polyethylenglycole oder andere wassermischbare Solventien.

Das "in Kontakt bringen" kann aber auch gleichzeitig mit der Einwirkung der Farbstoffzubereitung dadurch erfolgen, daß man die Keratinfaser mit der feinteiligen Metallverbindung vorbehandelt, so daß es bei der nachfolgenden Behandlung mit der Zubereitung der Oxidationsfarbstoffvorprodukte auf dem Haar zum Kontakt mit dem Oxidationskatalysator kommt.

Ein Zusatz chemischer Oxidationsmittel wie z. B. Wasserstoffperoxid oder Harnstoffperhydrat zur Zubereitung der Oxidationsfarbstoffvorprodukte ist überflüssig.

Als wasserunlösliche, nanoskalige Verbindungen eignen sich bevorzugt solche von Übergangsmetallen, die in verschiedenen Oxidationsstufen vorliegen können. Als wasserunlösliche Verbindungen gelten dabei solche, die in Wasser oder in dem wäßrigen Färbeeansatz bei 20°C zu weniger als 10⁻⁴ Gew.% (1 ppm), bezogen auf Metallionen, löslich sind. Bevorzugt geeignet sind vor allem die Oxide, insbesondere die Oxide des Kupfers, Kobalts, Eisens, Mangans, Molybdäns, Rheniums, Vanadiums und des Rutheniums. Aber auch gemischte Oxide, wie beispielsweise die Ferrite, sind erfindungsgemäß bevorzugt.

Verfahren zur Gewinnung von feinteiligen Metalloxiden mit Teilchengroßen im Nanometerbereich sind literaturbekannt, und solche Produkte sind kommerziell verfügbar. Geeignete Oxide können z.B. von Nanopowder Enterprises Inc. bezogen werden. Herstellverfahren für nanoskalige Metalloxide sind z.B. in EP 776 862 A1, WO 93/17959 oder EP 655 984 B1 beschrieben. Elektrochemische Verfahren sind z.B. aus Chem. Abstr. 110, 65662 (1988) und CA 114, 31811 (1990) bekannt. Ein Verfahren, das für die vorliegende Erfindung besonders gut geeignete Metalloxide liefert, ist in der deutschen Patentanmeldung DE-A1-198 40 842 beschrieben. Bei diesem Verfahren wird ein in einem organischen Elektrolyten gelöstes Salz des Metalls an einer Kathode elektrochemisch reduziert und in Gegenwart eines Oxidationsmittels, z.B. von gelöstem Sauerstoff, in-situ oxidiert. Dabei wird z.B. in Abhängigkeit vom O₂-Partialdruck, aus Cu⁺⁺-Salzen ein Gemisch aus Cu, CuO und Cu₂O erhalten, das eine Partikelgröße von 3-100 nm aufweist. Alternativ kann man auch eine Anode aus dem Metall, dessen Oxid man herstellen will, in einer organischen Elektrolytlösung anodisch lösen und an der Kathode in Gegenwart von Luftsauerstoff als ein feinteiliges Gemisch von Kupfer, Kupfer(I)-oxid und Kupfer(II)-oxid wieder abscheiden. Dieses Verfahren ist in Beispiel 1 näher beschrieben.

Die als Katalysator erforderliche Menge hängt im Einzelfall von der Art der Metallverbindung und von ihrer Feinteiligkeit, bzw. von ihrer spezifischen Oberfläche ab. Im allgemeinen dürfte eine Konzentration von wenigstens 1 ppm (berechnet als Metall und bezogen auf den gebrauchsfertigen Färbeansatz) erforderlich sein. Im konkreten Fall des unten angeführten Beispiels war eine Konzentration von 20 ppm eines Cu₂O/CuO/Cu-Gemisches mit einer Teilchengröße unter 100 nm (Nanometer) in dem gebrauchsfertigen Färbeansatz ausreichend, um eine volle Ausfärbung des Haars ohne Zusatz eines Oxidationsmittels, allerdings bei einem pH-Wert von 9, zu erzielen.

Daher scheint eine Teilchenfeinheit von 3-100 nm für die verwendete Metallverbindung besonders bevorzugt zu sein. Die optimale Konzentration der nanoskaligen Metallverbindung läßt sich anhand einiger Färbeversuche leicht ermitteln. Eine zu hohe Katalysatorkonzentration kann eine zu rasche und dann ungleichmäßige Farbentwicklung zur Folge haben.

Für die Zusammensetzung der wäßrigen Zubereitung der Oxidationsfarbstoff-Vorprodukte können die dafür üblichen Rohstoffe und Rezepturen mit pH-Werten von 5 bis 10 verwendet werden. Man kann aber den pH-Wert dieser Zusammensetzungen deutlich niedriger halten. In einer bevorzugten Ausführung weist die wäßrige gebrauchsfertige Zubereitung einen pH-Wert von 5 bis 8 auf. Dem Färbeansatz wird vor der Einwirkung auf die Keratinfasern kein Oxidationsmittel zugesetzt.

Es können zusätzlich weitere Oxidationskatalysatoren, z.B. Oxidaseenzyme wie Laccase, Tyrosinase oder Peroxidase eingesetzt werden.

Auch die wasserunlösliche feinteilige Metallverbindung wird bevorzugt erst unmittelbar vor der Einwirkung auf das Haar zum Färbeansatz gegeben. Die Anwendung auf dem Haar kann wie üblich mit einer Applikationsbürste oder aus einem Spendebehälter erfolgen. Auch die erforderliche Anwendetemperatur liegt wie üblich bei 15° bis 40°C, und die Einwirkungszeit kann zwischen 10 und 30 Minuten liegen. Nach der Einwirkung wird das überschüssige Haarfärbemittel durch Ausspülen und ggf. durch Nachwaschen mit einem Shampoo aus dem gefärbten Haar entfernt. Das Nachwaschen mit Shampoo kann entfallen, wenn ein stark tensidhaltiges Färbepräparat verwendet wurde.

Die nachfolgenden Beispiele sollen den Patentgegenstand näher erläutern:

### Beispiele

### 1. Elektrolytische Herstellung von nanoskaligern Cu₂O/CuO/Cu-Pulver (gemäß DE 19840842.0)

In 100 ml Tetralydrofuran mit einem Wassergehalt von 0,5-1 Gew.% wurde 1 g Tetrabutylammoniumbromid unter leichtem Erwärmen (40°C) gelöst. Als Anode wurde ein 20x40 mm großes und 1 mm dickes Kupferblech verwendet. Als Kathode diente ein 20x40 mm großes und 1 mm dickes Titanblech. Die Elektrolyse wurde bei einer Stromdichte von 3 mA/cm² über 24 Stunden unter leichter Bewegung (Rühren) des Elektrolyten bei ca. 35.40°C unter Durchleiten von Luft durchgeführt.

Dabei bildete sich zunächst eine.Trübung, dann ein Niederschlag der anschließend nach dem Absetzen abfiltriert und bei 100°C getrocknet wurde. Die analytische Untersuchung zeigte, daß es sich um ein Gemisch aus CuO und Cu₂O sowie metallischem Kupfer (zu etwa gleichenTeilen) handelte. Die mittlere Partikelgröße betrug 10-25 nm.

### 2. Zubereitung der Oxidationsfarbstoff Vorprodukte

### 2.1 Cremebasis

| | |
|---|---|
| Texapon® NS0 (Sodium Laureth Sulfate, 28 %ig) | : 20 Gew.% |
| Dehyton® K (Cocoamidopropyl Betaine, 30 %ig) | : 12,5 Gew.% |
| Lorol® technisch (Coconut C₁₂-C₁₈-Alcohol) | : 2,0 Gew.% |
| Hydrenol® D (Cetearyl Alcohol) | : 8,5 Gew.% |
| Eumulgin® B2 (Ceteareth 20) | : 0,75 Gew.% |

### 2.2 Haarfärbeemulsion

| | |
|---|---|
| Cremebasis (nach 2.1) | : 50,0 g |
| 4-Amino-2-aminomethyl-phenol | : 7,5 mmol |
| 5-Amino-2-methylphenol | : 7,5 mmol |
| Na₂SO₃ | : 1,0 g |
| (NH₄)₂SO₄ | : 1,0 g |
| konz. NH₃ Lösung in H₂O | : ad pH=9 |
| Wasser | : ad 100 g |

### 2.3 Oxidationsmittel

Wäßrige H₂O₂-Lösung (3%ig)

### 2.4 Katalysator-Dispersion

| | |
|---|---|
| Glycerin | :95,0g |
| Cu₂O/CuO/Cu-Pulver (nach 1) | : 5,0 g |

### 2.5 Färbeansätze

| **Färbeansätze** | **E1** | **E2** | **E3** | **E4** | **V1** | **V2** | **V3** |
|---|---|---|---|---|---|---|---|
| Haarfärbeemulsion (2,2) | 50 g | 50 g | 50 g | 50 g | 50 g | 50 g | 50 g |
| Oxidationsmittel (2,3) | - | - | - | - | 50 g | - | - |
| Wasser | 50 g | 50 g | 50 g | 50 g | - | 50 g | 50 g |
| Katalysator-Dispersion (2,4) | 0,4 g | 0,1 g | 0,04 g | 0,02 g - | - | - | - |
| Cu-Pulver | - | - | - | - | - | 0,4 g | - |
| CuO (Aldrich) < 5 µm | - | - | - | - | - | - | 0,4 g |

### 3. Färbeversuche

Der Färbeansatz wurde gemischt und auf ca. 5 cm lange Strähnen eines standardisierten Haars, Type Naturweiß (Fa. Kerling) aufgetragen und dort 30 Minuten bei ca. 30°C belassen. Danach wurde das Haar mit einem üblichen Shampoo gewaschen und getrocknet. Es wurden die folgenden Färbeergebnisse erhalten:

| | |
|---|---|
| E1 (200 ppm Katalysator) | : Vollausfärbung, zu rasche Entwicklung |
| E2 (50 ppm Katalysator) | : Vollausfärbung, zu rasche Entwicklung |
| E3 (20 ppm Katalysator) | : Vollausfärbung (vergleichbar mit V1) |
| E4 (10 ppm Katalysator) | : Keine Vollausfärbung |
| V1 (mit H₂O₂) | : Vollausfärbung (nicht erfindungsgemäß) |
| V2 (mit Cu-Pulver) | : Vollausfärbung, Farbe glanzlos, stumpf (nicht erfindungsgemäß) |
| V3 (mit CuO grobteilig) | : Keine Farbreaktion (nicht erfindungsgemäß) |

### 4. Färbeemulsionen

| | 4.1 | 4.2 |
|---|---|---|
| Cremebasis | 50 g | 50 g |
| p-Toluylendiamin | 2,5 m mol | - |
| m-Phenylendiamin | 2,5 m mol | - |
| p-Aminophenol | - | 2,5 m mol |
| 5-Amino-2-methylphenol | - | 2,5 m mol |
| Na₂ SO₃ | 1,0 g | 1,0 g |
| (NH₄)₂ SO₄ | 1,0 g | 1,0 g |
| Wasser und Alkalisierungsmittel ad pH=9,1 | ad 100 | ad 100 |

### 5. Färbeemulsionen

| | 5.1 | 5.2 | 5.3 | 5.4 |
|---|---|---|---|---|
| Cu₂O/CuO/Cu-Pulver (nach 1) | 0,004 | 0,010 | 0,02 | 0,04 |
| Wasser | ad 100 | ad 100 | ad 100 | ad 100 |

### 6. Färbeversuche

Es wurden Färbeansätze aus 50 g der Haarfärbeemulsionen 4.1 bzw. 4.2 und Wasser (Vergleich), 2 Gew.%-iger H₂O₂-Lösung (Vergleich) oder Katalysatordispersion 5.1 bis 5.4 hergestellt.

Die Färbeansätze wurden gemischt und auf ca. 5 cm lange Strähnen eines standardisierten Haars, Type Naturweiß (Fa. Kerling) aufgetragen und dort 30 Minuten bei ca. 30°C belassen. Danach wurde das Haar mit einem üblichen Shampoo gewaschen und getrocknet.

Das gefärbte Haar wurde dann colorimetrisch vermessen und die Farbstärke in % bezogen auf eine mit Wasserstoffperoxid anstelle des Katalysators durchgeführte Färbung (=100 %) berechnet.

Die Ergebnisse sind der folgenden Tabelle zu entnehmen. Man erkennt den Einfluß der Katalysatorkonzentration und der Art des Alkalisierungsmittels auf die oxidative Farbentwicklung.

| Alkalisierungsmittel | NH₃ | | HO-CH₂-CH₂-NH₂ | | NaOH | |
|---|---|---|---|---|---|---|
| Haarfärbeemulsion | 4.1 | 4.2 | 4.1 | 4.2 | 4.1 | 4.2 |
| Katalysator | | | | | | |
| Wasser (Vergleich) | 52% | 65% | 68% | 64% | 69% | 72% |
| 5.1 | 57% | 68% | 70% | 64% | 69% | 79% |
| 5.2 | 63% | 75% | 71% | 70% | 68% | 81% |
| 5.3 | 69% | 77% | 74% | 76% | 72% | 81% |
| 5.4 | 96% | 89% | 80% | 85% | 78% | 93% |
| wäßrige H₂O₂ Lösung (2%ig) Vergleich | 100% | 100% | 100% | 100% | 100% | 100% |

## Patentansprüche

1. Verwendung wasserunlöslicher, feinverteilter Verbindungen der Metalle der 3. bis 12. Gruppe des periodischen Systems einschließlich der Lanthanide, vorzugsweise der Oxide, mit einer Teilchengröße von weniger als 1000 nm, als Katalysatoren zur Farbentwicklung in Zubereitungen zur oxidativen Färbung von Haaren ohne Zusatz von Oxidationsmitteln.

2. Verfahren zur oxidativen Färbung von Keratinfasern unter Verwendung einer wässrigen Zubereitung von Oxidationsfarbstoffvorprodukten, die vor der Einwirkung auf die Fasern nicht mit einem Oxidationsmittel vermischt, dann mit dem Haar in Kontakt gebracht und nach einer Einwirkungszeit wieder ausgewaschen wird, **dadurch gekennzeichnet, dass** die Zubereitung unmittelbar vor oder gleichzeitig mit der Einwirkung auf die Keratinfasern mit einer wasserunlöslichen, feinteiligen Verbindung eines Metalls der 3. bis 12. Gruppe des periodischen Systems einschließlich der Lanthanide, vorzugsweise einem Oxid, mit einer Teilchengröße von weniger als 1000 nm in einer Menge von wenigstens 1 ppm (berechnet als Metall und bezogen auf den gebrauchsfertigen Färbeansatz), in Kontakt gebracht wird.

3. Verfahren nach Patentanspruch 2, **dadurch gekennzeichnet, dass** die Verbindung des Metalls ausgewählt ist aus den Oxiden von Kupfer, Kobalt, Eisen, Mangan, Molybdän, Rhenium, Vanadium und Ruthenium.

4. Verfahren nach einem der Ansprüche 2 oder 3, **dadurch gekennzeichnet, dass** die Metallverbindung eine Teilchengröße von 3-100 nm aufweist.

5. Verfahren nach einem der Ansprüche 2-4, **dadurch gekennzeichnet, dass** die wässrige, gebrauchsfertige Zubereitung einen pH-Wert von 5-8 aufweist.

6. Verfahren nach einem der Ansprüche 2-5, **dadurch gekennzeichnet, dass** die Metallverbindung im Gemisch mit feinteiligem Metall zugesetzt wird.

## Claims

1. Use of water-insoluble, finely divided compounds of metals from groups 3 to 12 of the Periodic Table of the Elements including the lanthanides, preferably the oxides, with a particle size of less than 1000 nm, as catalysts for developing colour in preparations for the oxidation dyeing of hair without the addition of oxidizing agents.

2. Method for the oxidation dyeing of keratin fibres using an aqueous preparation of oxidation dye precursors which, prior to being worked into the fibres, is not mixed with an oxidizing agent, then is brought into contact with the hair and, after a working-in time, is washed out again, **characterized in that** the preparation, immediately before or at the same time as working it into the keratin fibres, is brought into contact with a water-insoluble, finely divided compound of a metal from groups 3 to 12 of the Periodic Table of the Elements including the lanthanides, preferably an oxide, with a particle size of less than 1000 nm in an amount of at least 1 ppm (calculated as metal and based on the ready-to-use dyeing mixture).

3. Method according to patent Claim 2, **characterized in that** the compound of the metal is chosen from the oxides of copper, cobalt, iron, manganese, molybdenum, rhenium, vanadium and ruthenium.

4. Method according to one of Claims 2 or 3, **characterized in that** the metal compound has a particle size of 3-100 nm.

5. Method according to one of Claims 2-4, **characterized in that** the aqueous, ready-to-use preparation has a pH of 5-8.

6. Method according to one of Claims 2-5, **characterized in that** the metal compound is added in the mixture with finely divided metal.

## Revendications

1. Utilisation de composés insolubles dans l'eau, finement divisés, de métaux appartenant aux groupes 3 à 12 de la classification périodique des éléments, y compris les lanthanides, de préférence les oxydes, ayant une taille des particules inférieure à 1000 nm, en tant que catalyseurs pour le développement d'une couleur dans des préparations destinées à la coloration oxydante des cheveux sans addition d'agent oydant.

2. Procédé de coloration oxydante de fibres kératiniques utilisant une préparation aqueuse de précurseurs de colorants oxydants qui, avant l'action sur les fibres, n'est pas mélangée à un agent oxydant, ensuite mise en contact avec les cheveux et après une certaine durée d'action, éliminée par lavage, **caractérisé en ce que** la préparation est mise en contact, immédiatement avant ou simultanément à l'action sur les fibres kératiniques, avec un composé insoluble dans l'eau, finement divisé, d'un métal appartenant aux groupes 3 à 12 de la classification périodique des éléments, y compris les lanthanides, de préférence un oxyde, ayant une taille des particules inférieure à 1000 nm, en une quantité d'au moins 1 ppm (calculée en tant que métal et par rapport au mélange de teinture prêt à l'emploi).

3. Procédé selon la revendication 2, **caractérisé en ce que** le composé métallique est choisi parmi les oxydes de cuivre, de cobalt, de fer, de manganèse, de molybdène, de rhénium, de vanadium et de ruthénium.

4. Procédé selon l'une quelconque des revendications 2 ou 3, **caractérisé en ce que** le composé métallique présente une taille des particules de 3 à 100 nm.

5. Procédé selon l'une quelconque des revendications 2 à 4, **caractérisé en ce que** la préparation aqueuse prête à l'emploi présente un pH de 5 à 8.

6. Procédé selon l'une quelconque des revendications 2 à 5, **caractérisé en ce que** le composé métallique est ajouté en mélange avec du métal finement divisé.
